(19) Europäisches Patentamt
European Patent Office
Office européen des brevets

(11) Veröffentlichungsnummer: **0 042 358**
**A1**

(12) **EUROPÄISCHE PATENTANMELDUNG**

(21) Anmeldenummer: **81810232.9**

(22) Anmeldetag: **10.06.81**

(51) Int. Cl.³: **C 07 C 149/23**, C 07 C 149/20, C 07 D 263/14, C 08 K 5/36

(30) Priorität: **16.06.80 CH 4615/80**

(43) Veröffentlichungstag der Anmeldung: **23.12.81 Patentblatt 81/51**

(84) Benannte Vertragsstaaten: **DE FR GB IT**

(71) Anmelder: **CIBA-GEIGY AG, Patentabteilung Postfach, CH-4002 Basel (CH)**

(72) Erfinder: **Rosenberger, Siegfried, Im Gehracker 11, CH-4125 Riehen (CH)**
Erfinder: **Evans, Samuel, Dr., Schützenrain 3, CH-4125 Riehen (CH)**

(54) **Organische Sulfide und ihre Verwendung als Stabilisatoren in organischen Materialien.**

(57) Die erfindungsgemässen Stabilisatoren entsprechen den folgenden Formeln I oder II

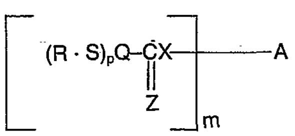

(I)

Darin bedeuten m eine Zahl zwischen 1 und 12, p eine Zahl zwischen 1 und 3, R beispielsweise Alkyl, A beispielsweise bei m = 1 den Rest

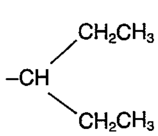

X und Z z. B. jeweils –O– und Q z. B. bei p = 1 $-CH_2-$.

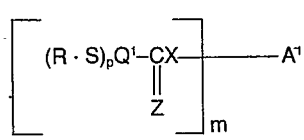

(II)

Darin haben R, $Q^1$, X, Z und p beispielsweise die oben bei Formel I angegebene Bedeutung. $A^1$ kann z. B. einen Alkylenrest mit 2 bis 20 C-Atomen und m 2, 3 oder 4 bedeuten.

Die Verbindungen sind insbesondere als Stabilisatoren oder Co-Stabilisatoren für Polymere und Schmiermittel geeignet.

EP 0 042 358 A1

CIBA-GEIGY AG

Basel (Schweiz)

3-12904/+

Organische Sulfide und ihre Verwendung als Stabilisatoren in organischen Materialien

Die Erfindung betrifft neue organische Verbindungen, welche -S-Brückenglieder enthalten, und ihre Verwendung als Stabilisatoren für organisches Material.

Es ist bereits durch die DE-OS 2 028 240 bekannt, Polyolefine durch bekannte -S-Brücken enthaltende Ester gegen thermo-oxidativen Abbau zu stabilisieren. Derartige Stabilisatoren sind aber durchaus nicht optimal in ihrer Wirkung, da entsprechende Zusammensetzungen bei der thermo-oxidativen Belastung nicht farbkonstant sind und die Extraktionsstabilität leidet.

In dem US-Patent 3 758 549 werden -S-Brücken enthaltende, mehrwertige Carbonsäureester beansprucht, welche überwiegend in Kombination mit phenolischen Antioxidantien als Stabilisatoren gegen thermooxidativen Abbau in organischem Material verwendet werden. Diese S-haltigen Stabilisatoren gemäss dem US-Patent 3 758 549 weisen dieselben Nachteile wie die oben beschriebenen bekannten -S-Brücken enthaltenden Ester auf.

In der US-PS 4 125 515 werden ebenfalls -S-Brücken enthaltende Ester beschrieben. Dieselben werden aber ausschliesslich in Kombination mit organischen Aminen als Antioxidantien verwendet. Dabei beschränkt sich die Verwendung auf SBR- und NBR-Polymere, d.h. auf elastomere Polymere. Die so verwendeten S-haltigen Ester weisen bei der Verwendung als Stabilisatoren ohne den zusätzlichen Einsatz von organischen Aminen dieselben Nachteile wie die gemäss dem vorher besprochenen Stand der Technik verwendeten Stabilisatoren auf.

Die erfindungsgemässen, -S-Brücken enthaltenden organischen Verbindungen weisen die Nachteile der bekannten S-haltigen Stabilisatoren nicht auf. Sie stabilisieren das organische Material besser in Bezug auf Farbe und Extraktionseigenschaften und zeigen als Co-Stabilisatoren ausgeprägten Synergismus.

Gegenstand der Erfindung sind Verbindungen der Formel I

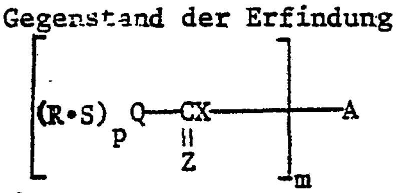 (I)

in der X einen der Reste -O-, -NH- oder $-NR^1-$ bedeutet, wobei beim Vorliegen mehrerer Reste X dieselben gleich oder verschieden sind, Z einen der Reste -O- oder -S-, m eine Zahl zwischen 1 und 12 und p eine ganze Zahl zwischen 1 und 3 bedeuten, wobei $R^1$ für einen geradkettigen oder verzweigten Alkylrest mit 1 bis 25 C-Atomen steht, in der R einen geradkettigen oder verzweigten, gesättigten oder ungesättigten Alkylrest mit 1 bis 30 Kohlenstoffatomen, welcher durch Aryl-, Aminocarbonyl-, Alkylaminocarbonyl- oder OH-Gruppen substituiert und durch eine -CO•O-, -O•CO-, -O- oder -S-Brücke unterbrochen sein kann, darstellt, in der Q einen (p+1)-wertigen geradkettigen oder verzweigten, gesättigten oder ungesättigten Kohlenwasserstoffrest mit 1 bis 20 Kohlenstoffatomen oder einen Rest der Formel 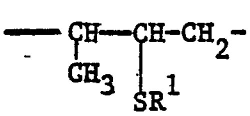 oder den 3-wertigen Rest 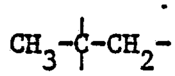 bedeutet, und

in der A

a) einen 1-wertigen Rest 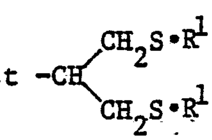,

oder

b) einen 2-wertigen Rest aus der Gruppe $-(C_nH_{2n}O)_q-C_nH_{2n}-$,

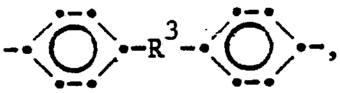, $-R^3-CO\cdot O-R^3-$, $-R^3-CH=CH-R^3-$, $-R^3-C\equiv C-R^3-$,

$-R^3-NH\cdot CO\cdot CO\cdot NH-R^3-$, 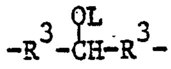, $-R^3-NH\cdot CO\cdot NH-R^3-$, 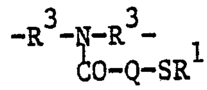 ,

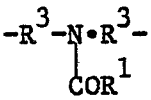 , 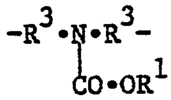 , 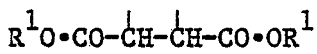, 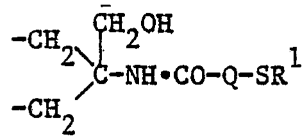,

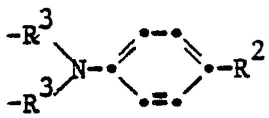 und 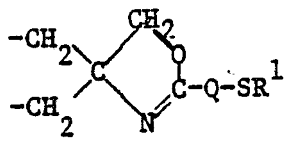,

wobei $R^2$ die Definition von $R^1$ hat, Wasserstoff bedeutet oder

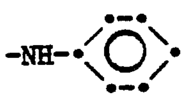 darstellt,

n eine ganze Zahl zwischen 3 und 6, q eine Zahl zwischen 1 und 20, $R^3$ einen geradkettigen oder verzweigten, gesättigten oder ungesättigten Alkylenrest mit 1 bis 20 C-Atomen und L Wasserstoff oder $-CO\cdot R^1$ bedeuten, oder

c) einen der 3-wertigen Reste

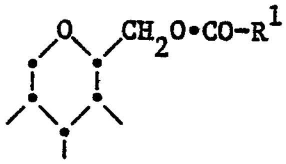 , 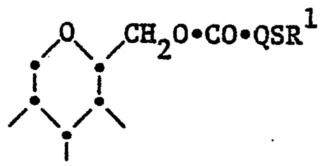 , 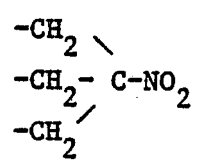

und 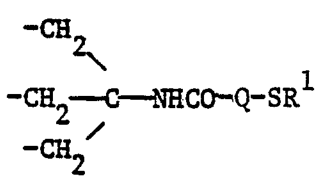

bedeutet, oder

d) einen der 4-wertigen Reste 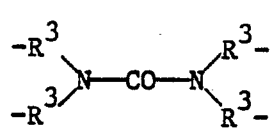 ,

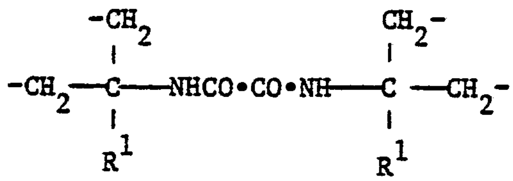 , 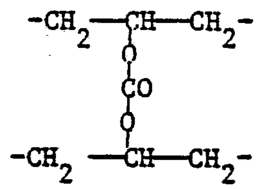 ,

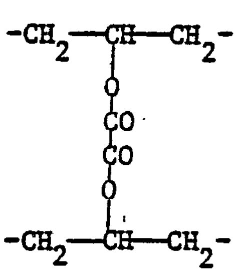 und 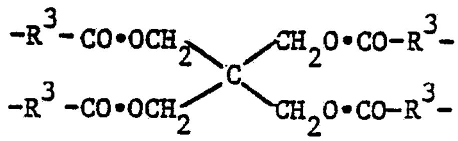

bedeutet, oder

e) einen der 6-wertigen Reste

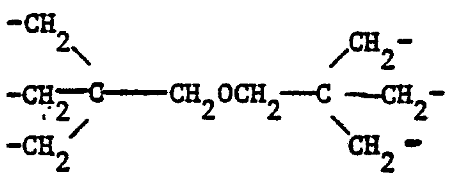 ,

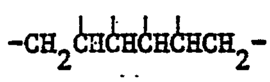 und 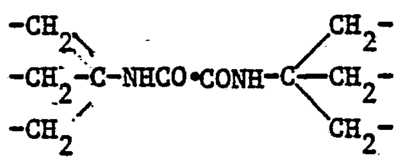

bedeutet oder

f) den 8-wertigen Rest

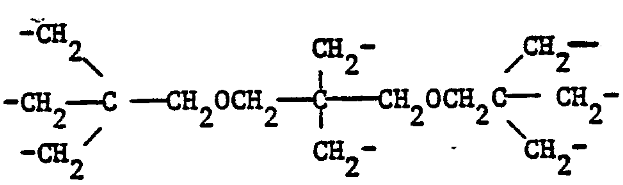 darstellt,

in der aber für den Fall, dass X einen der Reste -NH- oder $-NR^1-$ darstellt und teilweise aber nicht ausschliesslich auch -O- bedeuten kann, A auch für einen 2-wertigen, geradkettigen oder verzweigten, gesättigten oder ungesättigten Kohlenwasserstoffrest mit 2 bis 18 C-Atomen oder für einen 3- oder 4-wertigen,

geradkettigen oder verzweigten, gesättigten oder ungesättigten Kohlenwasserstoffrest mit 3 bis 18 C-Atomen stehen kann, und Verbindungen der Formel II

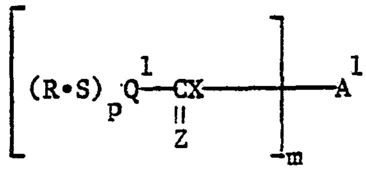 (II) ,

in der X einen der Reste -O-, -NH- oder $-NR^1-$ bedeutet, wobei beim Vorliegen mehrerer Reste X dieselben gleich oder verschieden sind, Z einen der Reste -O- oder -S-, m die Zahl 2, 3 oder 4 und p eine ganze Zahl zwischen 1 und 3 bedeuten, wobei $R^1$ für einen geradkettigen oder verzweigten Alkylrest mit 1 bis 25 C-Atomen steht, in der R einen gesättigten oder ungesättigten Alkylrest mit 1 bis 30 Kohlenstoffatomen, welcher durch Aryl-, Aminocarbonyl-, Alkylaminocarbonyl- oder OH-Gruppen substituiert und durch eine -CO•O-, -O•CO-, -O- und -S-Brücke unterbrochen sein kann, darstellt, in der $Q^1$ den Rest $-CH_2-$ oder einen Rest der Formel 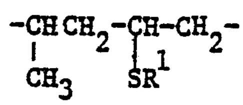 oder den 3-wertigen Rest 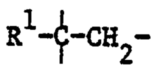 bedeutet, und in der $A^1$ für einen 2-wertigen, geradkettigen oder verzweigten, gesättigten oder ungesättigten Kohlenwasserstoffrest mit 2 bis 20 C-Atomen oder für einen 3- oder 4-wertigen, geradkettigen oder verzweigten, gesättigten oder ungesättigten Kohlenwasserstoffrest mit 3 bis 18 C-Atomen steht.

Ueber die in den Formeln I und II angegebenen Reste kann im einzelnen noch folgendes ausgeführt werden.

$R^1$ kann folgende Alkylreste bedeuten: z.B. Methyl, iso-Propyl, n-Butyl, sec.-Butyl, tert.-Butyl, tert.-Amyl, 2-Aethylhexyl, n-Octyl, tert.-Octyl, n-Dodecyl, 1,1,7,7-Tetramethyl-octyl, n-Octadecyl, α-verzweigte Alkyl-Radikale mit 3 bis 8 C-Atomen sind bevorzugt.

R kann dieselben Reste wie $R^1$ darstellen, wobei R und $R^1$ in Formel I aber nicht identisch zu sein brauchen. Weiter kann R beispielsweise auch n-Docosyl, n-Triacontyl, Oleyl und Allyl bedeuten.

Q kann z.B. Methylen, Aethylen, Propylen, iso-Propylen, n-Octylen, 2-(Prop-1-enyl)-äthylen und n-Heptadecylen darstellen.

$R^3$ hat die gleiche Definition wie Q, soweit Q als 2-wertiger geradkettiger oder verzweigter, gesättigter oder ungesättigter Kohlenwasserstoffrest mit 1 bis 20 Kohlenstoffatomen definiert ist. Beide Reste brauchen in Formel I aber nicht dieselbe Bedeutung zu haben.

L kann beispielsweise Acetyl und Stearyl bedeuten.

$A^1$ kann entweder die Bedeutung von $R^3$ haben (wobei beide Reste nicht identisch zu sein brauchen), oder $A^1$ steht beispielsweise für die folgenden Reste:

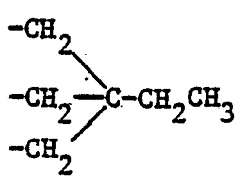

oder

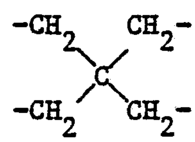

.

Die oben definierten Verbindungen der Formel I stellen eine Vorzugsform der Erfindung dar.

Bevorzugt bedeutet X in Formel I -O-.

Besonders bevorzugte erfindungsgemässe Verbindungen sind solche der Formel I, in der A

a) einen 1-wertigen Rest 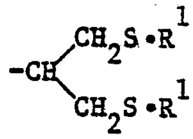 ,

oder

b) einen 2-wertigen Rest aus der Gruppe $-(C_nH_{2n}O)_q-C_nH_{2n}-$,

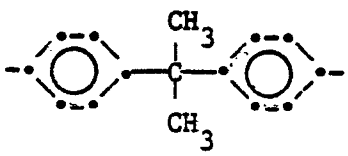 , 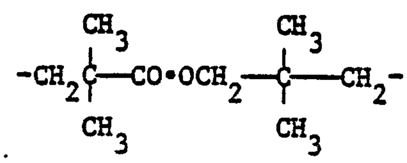 , $-CH_2-C\equiv C-CH_2-$ ,

$-CH_2CH=CHCH_2-$, 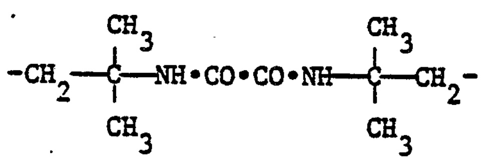 , 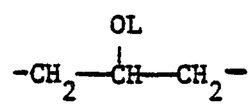 ,

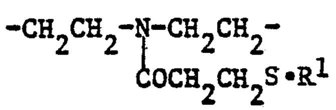 , $-CH_2CH_2-NH-CO-NH-CH_2CH_2-$,

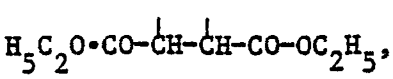, 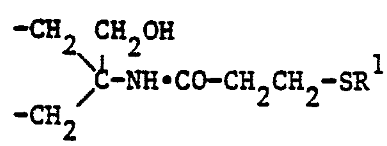 ,

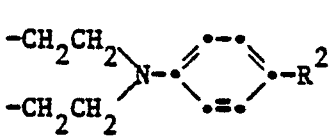 und 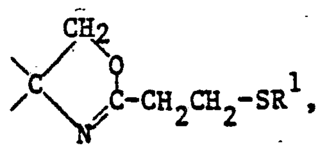,

wobei $R^2$ die Definition von $R^1$ hat, Wasserstoff bedeutet oder

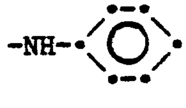 darstellt,

n eine ganze Zahl zwischen 3 und 6, q eine Zahl zwischen 1 und 20 und L Wasserstoff oder $-CO\cdot R^1$ bedeuten, oder

c) einen der 3-wertigen Reste

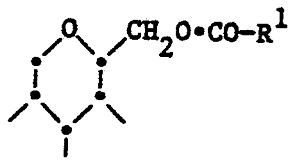 , 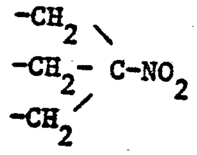 und 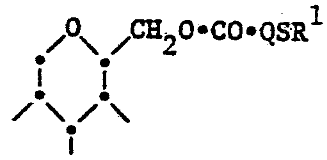

bedeutet, oder

d) einen der 4-wertigen Reste 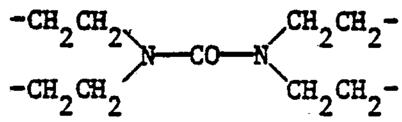 ,

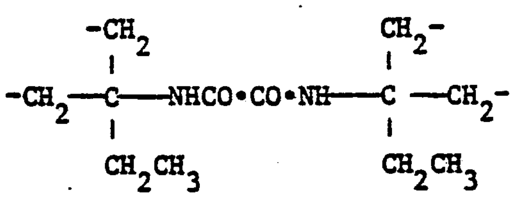 , 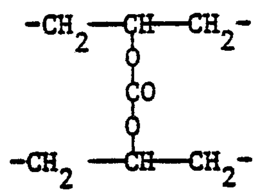 ,

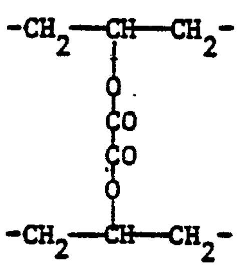 und 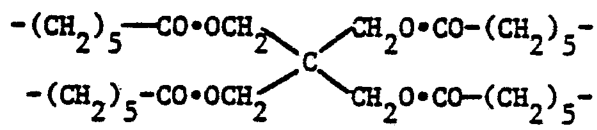

bedeutet, oder

e) einen der 6-wertigen Reste

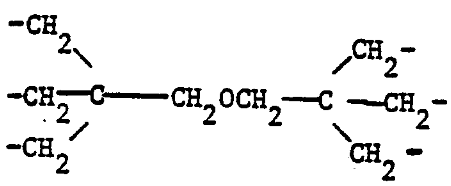 ,

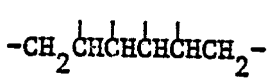 und 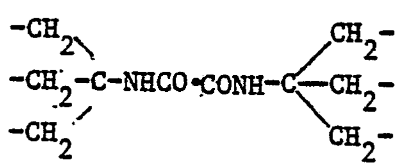

bedeutet oder

f) den 8-wertigen Rest

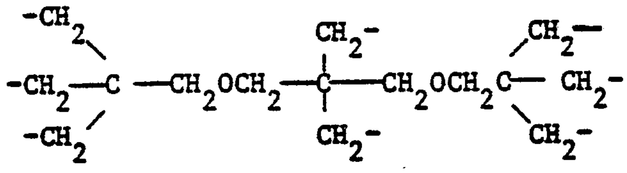 darstellt.

Eine speziell bevorzugte erfindungsgemässe Verbindung ist das Hexa-3-n-dodecylmercaptopropionyl-dipentaerythrit.

Bevorzugte erfindungsgemässe Verbindungen sind auch solche der Formel II, in der mindestens ein Rest X -O- bedeutet.

Als Beispiele für Verbindungen der Formel I sind Substanzen der folgenden Strukturen anzuführen:

$[(n\text{-}C_{12}H_{25}SCH_2CH_2COO)_3C\text{-}CH_2\text{-}]_2{=}O$

$n\text{-}C_{12}H_{25}SCH_2CH_2\underset{\overset{\|}{O}}{C}NHCH_2CH_2O\underset{\overset{\|}{O}}{C}CH_2CH_2S\text{-}n\text{-}C_{12}H_{25}$

$(n\text{-}C_{12}H_{25}SCH_2CH_2\underset{\overset{\|}{O}}{C}NHCH_2)_2$

$(n\text{-}C_{12}H_{25}\text{-}S\text{-}CH_2CH_2\underset{\overset{\|}{O}}{C}NHCH_2CH_2CH_2)_2$

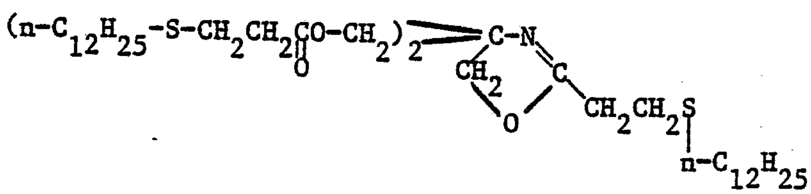

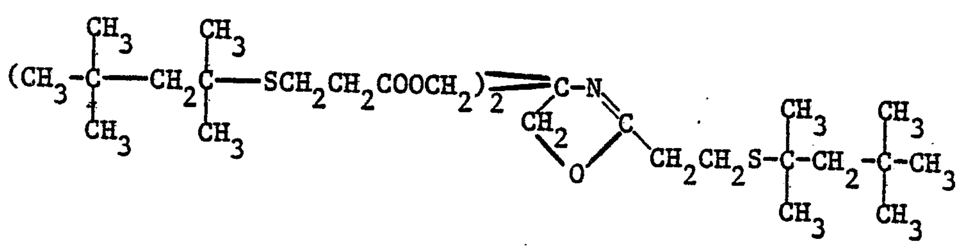

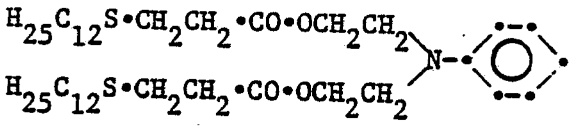

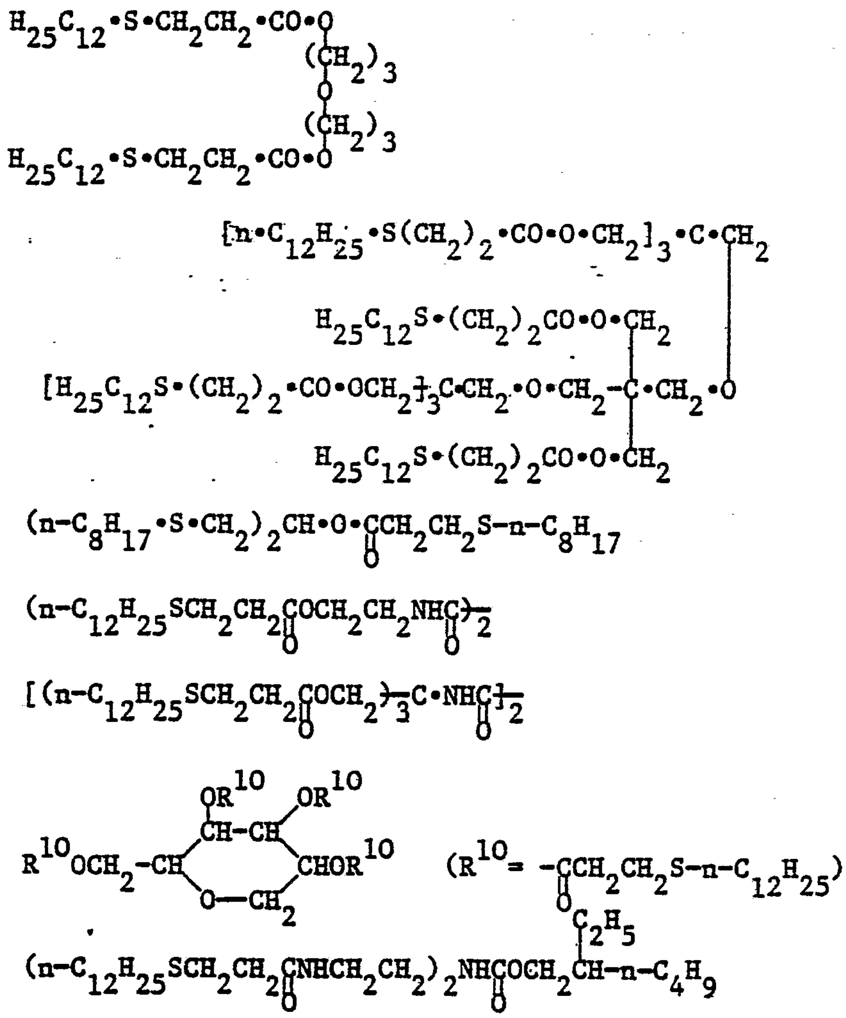

Als Beispiele für Verbindungen der Formel II sind Substanzen der folgenden Strukturen zu nennen:

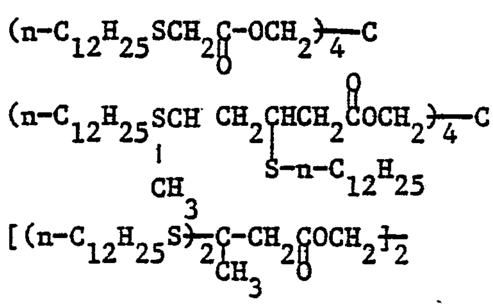

Die Herstellung der erfindungsgemässen Verbindungen kann nach verschiedenen an sich bekannten Verfahren erfolgen und sie erfolgt auch unterschiedlich, je nachdem, welche Bedeutung in den Formeln I und II X und Z haben.

Stellen X und Z jeweils beide Sauerstoff dar und handelt es sich somit bei den Verbindungen der Formeln I und II um Ester, so sind für die Herstellung derselben alle die Arbeitsweisen, bei denen Estergruppen nach Formel I bzw. II aufgebaut werden, anwendbar. Das sind folgende Methoden:

a) Direktveresterung einer Säure $(RS-)_pQ-CO.OH$ mit einem Alkohol $(HO-)_mA$ (analog jeweils für Verbindungen der Formel II mit $Q^1$ und $A^1$)

b) Umesterung eines Esters $(RS-)_pQ-CO.O$-niedriges Alkyl mit einem Alkohol $(HO-)_mA$

c) Reaktion eines Säurehalogenids $(RS-)_pQ-CO-Hal$ (Bevorzugt Cl) mit einem Alkohol $(HO-)_mA$.
Dabei wird vorzugsweise in Gegenwart der äquivalenten Menge einer Halogenwasserstoff bindenden Base (anorganische Base oder organisches tertiäres Amin) gearbeitet.

Die Reaktionen a) bis c) stellen bekannte Verfahren dar. Sie werden entweder in der Schmelze, bevorzugt bei erhöhter Temperatur bis etwa 180°C (vorzugsweise bei 50-150°C), durchgeführt oder man arbeitet in Lösung, d.h. man verwendet inerte Lösungsmittel, wie Alkane, Halogenalkane, aromatische Kohlenwasserstoffe, Aether und dergleichen.

Es kann aber auch von Ausgangsverbindungen ausgegangen werden, welche die typischen Estergruppen nach Formel I bzw. II bereits enthalten. So ist z.B. eine Additionsreaktion durchführbar, welche nach dem folgenden verallgemeinerten Reaktionsschema verläuft:

d) $$RSH + (\underbrace{CH_2{=}CH{-}C_wH_{2w}}_{Q}{-}\underset{\underset{Z}{\|}}{C}X{-})_mA \longrightarrow [(RS-)_pQ{-}\underset{\underset{Z}{\|}}{C}X{-}]_mA$$

(w = 0-28)

Diese Reaktion wird je nachdem, wie weit aktiviert die Doppelbindungen sind, ionisch oder radikalisch katalysiert. Geeignete Katalysatoren sind die folgenden.

Ionische: Tertiäre organische Basen, wie Trialkylamine, Pyridin, N-Alkylpiperidine, anorganische Basen, wie Alkalialkoholate, -hydroxide und -carbonate.

Radikalische: Azoverbindungen, wie Azodiisobutyronitril, Peroxide, wie $H_2O_2$, Dibenzoylperoxid, Cumolhydroperoxid.

Bedeutet in Formel I Z -S- und X Sauerstoff, so sind praktisch analoge Verfahren gemäss a) bis d) anwendbar. Bevorzugt ist in diesem Falle das c) -analoge Verfahren.

Wenn X in Formel I einmal, mehrmals oder ausschliesslich -NH- oder $-NR^1-$ bedeutet, so wird die Acylierung am Stickstoff nach bekannten Verfahren analog den Methoden a) bis c), vorzugsweise nach Methode b) und unter Einsatz der entsprechenden Ausgangsstoffe vorgenommen. Für den Fall, dass X sowohl -O- als auch $-NH-(-NR^1-)$ bedeutet, kann die Acylierungsreaktion ohne weiteres gleichzeitig an N und O vorgenommen werden. Methode d) ist ebenfalls analog anwendbar.

Die als Ausgangssubstanzen für die Herstellung der erfindungsgemässen Verbindungen der Formeln I und II verwendeten speziellen Säuren, Ester, Alkohole und Säurehalogenide sind bekannte Stoffe, deren Herstellung dem Fachmann ebenfalls bekannt ist.

Weiterer Gegenstand der Erfindung ist die Verwendung der erfindungsgemässen Verbindungen der Formel I und/oder II als Stabilisatoren oder Co-Stabilisatoren für organisches Material. Die Stabilisatorwirkung betrifft im wesentlichen den thermo-oxidativen Abbau. Die erfindungsgemässen Verbindungen führen in der Anwendung in

Kombination mit anderen Stabilisatoren, bevorzugt mit Phenol-Antioxidantien, zu stark ausgeprägten synergistischen Effekten. Es kann praktisch jegliches abbaugefährdetes technisches organisches Material, wie Kunststoffe, Schmiermittel, Hydraulikflüssigkeiten und dergleichen stabilisiert werden. Ausführlicher sind im einzelnen die folgenden gemäss der Erfindung stabilisierbaren Substrate anzuführen:

1. Polymere von Mono- und Diolefinen, beispielsweise Polyäthylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z.B. von Cyclopenten oder Norbornen.

2. Mischungen der unter 1) genannten Polymeren, z.B. Mischungen von Polypropylen mit Polyäthylen oder mit Polyisobutylen.

3. Copolymere von Mono- und Diolefinen untereinander oder mit anderen Vinylmonomeren, wie z.B. Aethylen-Propylen-Copolymere, Propylen-Buten-1-Copolymere, Propylen-Isobutylen-Copolymere, Aethylen-Buten-1-Copolymere, Propylen-Butadien-Copolymere, Isobutylen-Isopren-Copolymere, Aethylen-Aethylacrylat-Copolymere, Aethylen-Alkylmethacrylat -Copolymere, Aethylen-Vinylacetat-Copolymere oder Aethylen-Acrylsäure-Copolymere und deren Salze (Ionomere), sowie Terpolymere von Aethylen mit Propylen und einem Dien, wie Hexadien, Dicyclopentadien oder Aethylidennorbornen.

4. Polystyrol.

5. Statistische Copolymere von Styrol oder α-Methylstyrol mit Dienen oder Acrylderivaten, wie z.B. Styrol-Butadien, Styrol-Acrylnitril, Styrol-Aethylmethacrylat, Styrol-Acrylnitril-Methacrylat; Mischungen von hoher Schlagzähigkeit aus Styrol-Copolymeren und einem anderen Polymer wie z.B. einem Polyacrylat, einem Dien-Polymeren oder einem Aethylen-Propylen-Dien-Terpolymeren; sowie Block-Copoly-

mere des Styrols, wie z.B. Styrol-Butadien-Styrol, Styrol-Isopren-Styrol, Styrol-Aethylen/Butylen-Styrol oder Styrol-Aethylen/Propylen-Styrol.

6. Pfropfcopolymere von Styrol, wie z.B. Styrol auf Polybutadien, Styrol und Acrylnitril auf Polybutadien, Styrol und Alkylacrylate, bzw. Alkylmethacrylate auf Polybutadien, Styrol und Acrylnitril auf Aethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS, MBS, ASA oder AES-Polymere bekannt sind.

7. Halogenhaltige Polymere, wie z.B. Polychloropren, Chlorkautschuk, chloriertes oder chlorsulfoniertes Polyäthylen, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Vinylchlorid-Copolymere, oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallylphthalat, Polyallylmelamin.

0042358

11. Homo- und Copolymere von cyclischen Aethern, d.h. Polyalkylenglykole, wie Polyäthylenoxyd, Polypropylenoxyd oder deren Copolymere mit Bisglycidyläthern.

12. Polyacetale, wie Polyoxymethylen, sowie solche Polyoxymethylene, die Comonomere wie z.B. Aethylenoxyd enthalten.

13. Polyphenylenoxyde und -sulfide.

14. Polyurethane, die sich von Polyäthern, Polyestern und Polybutadienen mit endständigen Hydroxylgruppen einerseits und aliphatischen oder aromatischen Polyisocyanaten andererseits ableiten sowie deren Vorsprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalamid, Poly-m-phenylen-isophthalamid, sowie deren Copolymere mit Polyäthern wie z.B. mit Polyäthylenglykol, Polypropylenglykol oder Polytetramethylenglykol.

16. Polyharnstoffe, Polyimide und Polyamid-imide.

17. Polyester, die sich von Dicarbonsäuren und Dialkoholen und/oder von Hydroxycarbonsäuren oder den entsprechenden Lactonen ableiten, wie Polyäthylenterephthalat, Polybutylenterephthalat, Poly-1,4-dimethylolcyclohexanterephthalat, Polyhydroxybenzoate, sowie Block-Polyäther-ester, die sich von Polyäthern mit Hydroxylendgruppen ableiten.

18. Polycarbonate.

19. Polysulfone und Polyäthersulfone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycidyläthern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseäther, wie Methylcellulose.

27. Natürliche und synthetische organische Stoffe, die reine monomere Verbindungen oder Mischungen von solchen darstellen, beispielsweise Mineralöle, tierische und pflanzliche Fette, Oele und Wachse, oder Oele, Wachse und Fette auf Basis synthetischer Ester

(z.B. Phthalate, Adipate, Phosphate oder Trimellithate), sowie Abmischungen synthetischer Ester mit Mineralölen in beliebigen Gewichtsverhältnissen, wie sie z.B. als Weichmacher für Kunststoffe oder als Spinnpräparationen Anwendung finden, sowie deren wässrigen Emulsionen.

28. Wässrige Emulsionen natürlicher oder synthetischer Kautschuke wie z.B. Naturkautschuk-Latex oder Latices von carboxylierten Styrol-Butadien-Copolymeren.

Bevorzugt betrifft die erfindungsgemässe Verwendung die Stabilisierung von organischen Polymeren, insbesondere von Polyolefinen, und Schmiermitteln (synthetische und natürliche). Die Konzentration der erfindungsgemässen Stabilisatoren in dem organischen Material liegt vorzugsweise zwischen 0,005 und 10 Gew.-%, insbesondere zwischen 0,05 und 1,5 Gew.-%, bezogen auf die gesamte stabilisierte Mischung.

Als Co-Stabilisatoren für die erfindungsgemässen Stabilisatoren kommen insbesondere phenolische Stabilisatoren in Frage. Zahlreiche solcher Co-Stabilisatoren sind in der US-PS 3 758 549 in den Spalten 2 bis 6 aufgezählt. Auch Phenole vom Typ

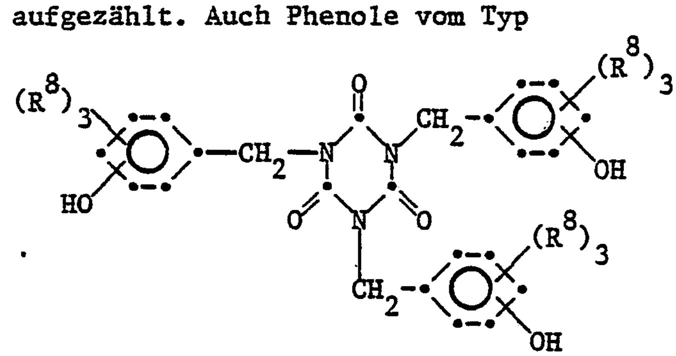
,

wobei $R^8$ $C_{1-12}$-Alkyl (geradkettig oder verzweigt) oder H bedeutet und mindestens ein Rest $R^8$ in o-Stellung zu -OH steht, sind geeignet. Besonders gut geeignete Co-Stabilisatoren gemäss der Erfindung sind Antioxidantien der Formeln

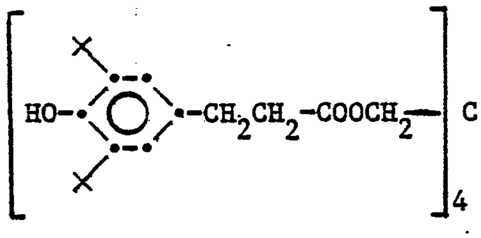 und 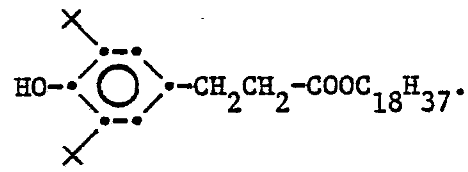

Die Konzenstration der Co-Stabilisatoren für die erfindungsgemässen Stabilisatoren liegt zweckmässig zwischen 0,01 und 5 %, vorzugsweise zwischen 0,1 und 0,5 %, bezogen auf die gesamte stabilisierte Mischung.

Bei dem Einsatz der erfindungsgemässen Verbindungen als Co-Stabilisatoren tritt ein beachtenswerter synergistischer Effekt bezüglich der Stabilisierung auf, welcher den Synergismus von bekannten kombinierten Antioxidantien übertrifft.

Weiterer Gegenstand der Erfindung ist auch noch das durch die erfindungsgemässen Verbindungen oder durch Gemische von den erfindungsgemässen Verbindungen mit phenolischen Stabilisatoren stabilisierte organische Material.

Herstellungs-Beispiele

Beispiel 1:

Hexa-3-n-dodecylmercapto-propionyl-dipentaerythrit

$$[(n\text{-}C_{12}H_{25}SCH_2CH_2COO)_3C\text{-}CH_2\text{-}]_2= O$$

28,8 g (0,1 Mol) 3-n-Docecylmercaptopropionsäuremethylester und 3,8 g (0,015 Mol) Dipentaerythrit werden unter Zugabe von 0,3 g Na-methylat 14 Stunden unter $N_2$ und Rühren auf 150-165°C erhitzt. Dabei können ca. 0,09 Mol abgespaltenes Methanol in einer Kühlfalle bei -20°C aufgefangen werden.

Die fast farblose Schmelze wird nach dem Abkühlen mit 100 ml Toluol und einigen Tropfen Essigsäure versetzt, die Lösung mit Wasser ausgeschüttelt und über $Na_2SO_4$ getrocknet.

Nach Entfernen des Lösungsmittels im Vakuum bleibt ein farbloses viskoses Oel zurück, aus welchem sich beim Digerieren mit einem Gemisch aus je 1 Teil Aceton und Methanol der kristalline Hexaester vom Smp. 45-50°C abscheidet.

Beispiele 2-4: Ersetzt man in Beispiel 1 den Dipentaerythrit durch Aethanolamin (Beispiel 2) oder Aethylendiamin (Beispiel 3) oder Hexamethylendiamin (Beispiel 4), so erhält man bei Verwendung aliquoter Mengen des 3-n-Dodecylmercapto-propionsäuremethylesters und unter Reaktionsbedingungen von 4-6 Stunden bei 150-160°C bei Verwendung von LiH als Katalysator die kristallinen Acylierungsprodukte der folgenden Formeln

(Beispiel 2) $n-C_{12}H_{25}SCH_2CH_2\underset{\overset{\|}{O}}{C}NHCH_2CH_2O\underset{\overset{\|}{O}}{C}CH_2CH_2S-n\cdot C_{12}H_{25}$ Smp. 83°C;

(Beispiel 3) $(n-C_{12}H_{25}SCH_2CH_2\underset{\overset{\|}{O}}{C}NHCH_2)_2$ Smp. 144°C;

(Beispiel 4) $(n-C_{12}H_{25}-S-CH_2CH_2\underset{\overset{\|}{O}}{C}NHCH_2CH_2CH_2)_2$ Smp. 134°C;

Beispiel 5:

2-(n-Dodecylmercapto-äthyl)-4,4-bis-(3-n-dodecylmercapto-propionyloxymethyl)-2-oxazolin

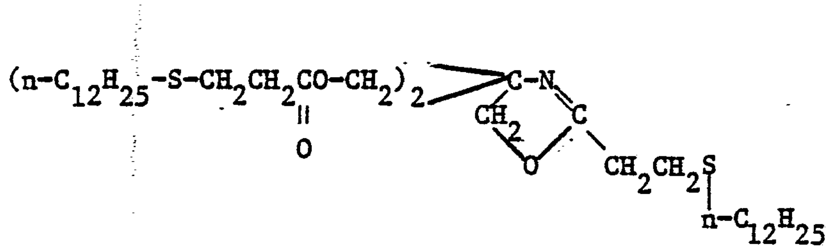

49 g (0,17 Mol) 3-n-Dodecylmercapto-propionsäuremethylester und 6,05 g (0,05 Mol) Tris-methylol-aminomethan werden unter Zugabe von 0,2 g LiH 15 Stunden unter $N_2$ und Rühren auf 160° erhitzt. Die schwach gelbliche Schmelze wird wie in Beispiel 1 beschrieben aufgearbeitet. Das Oxazolin kristallisiert beim Digerieren mit Acetonitril. Smp. ca. 45°C.

Beispiel 6:

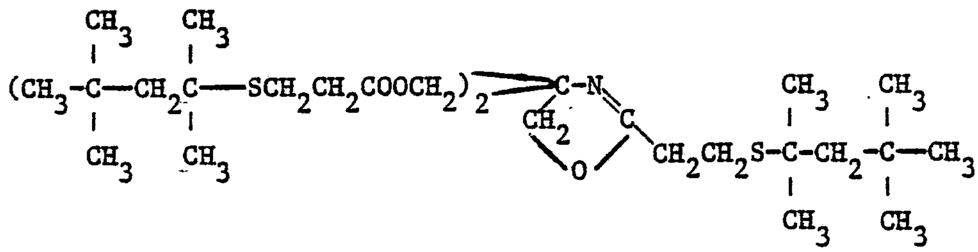

Ersetzt man in Beispiel 5 den n-Dodecylmercaptopropionsäuremethylester durch das ensprechende 1,1,3,3-Tetramethyl-n-butylmercapto-Derivat so erhält man bei analoger Arbeitsweise das entsprechende Oxazolin der angegebenen Struktur als fast farbloses viskoses Oel.

Beispiel 7:

N,N-Bis[3-n-dodecylthiopropionyloxyethyl]-anilin.

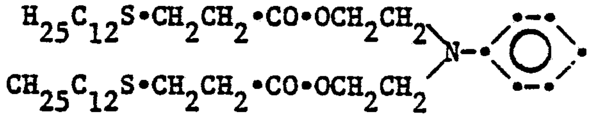

Bei 70°C werden 40,3 g (0,195 Mol) N-Phenyldiethanolamin, 115,2 g (0,39 Mol) Dodecylmercaptopropionsäure-methylester gelöst und 1 ml Tetrabutylorthotitanat monomer zugegeben. Unter Hochvakuum

0042358

wird das Reaktionsgemisch auf 120°C erhitzt und das entstehende Methanol abdestilliert. Nach 5 Stunden wird das Gemisch mit ca. 200 ml Methanol gewaschen und anschliessend aus 200 ml Isopropanol umkristallisiert. Das Produkt fällt zu 89,8 g in Form leicht beiger Kristalle an. Smpkt. 50°C.

Beispiel 8:

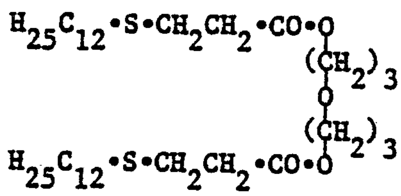

Bei 80°C unter $N_2$ werden zu 13,4 g (0,1 Mol) Dipropylenglykol und 57,7 g (0,2 Mol) Dodecylmercaptopropionsäure-methylester 0,5 g LiH zugegeben. Innerhalb von 16 Stunden steigt die Temperatur bis 155-160°C. Das entstandene Methanol wird abdestilliert. Danach wird das Reaktionsgemisch mit ca. 150 ml Toluol verdünnt, das LiH mit 2n HCl neutralisiert und das Lösungsmittel abgedampft. Der Rückstand besteht aus 62,4 g eines klaren, hell gelben Oels.

Beispiel 9:

N,N'-Di-n-dodecylmercaptopropionyloxyäthyl-oxamid

$(n\text{-}C_{12}H_{25}SCH_2CH_2COOCH_2CH_2NH\text{-}CO\text{-})_2$

28,8 g n-Dodecylmercaptopropionsäure-methylester und 8,8 g N,N'-Dihydroxyäthyl-oxamid [1] werden unter Zugabe von 0,5 g Tetrabutyl-ortho-titanat 4 Stunden unter $N_2$ mit Rühren auf 160°C erhitzt. Das Reaktionsprodukt wird aus Acetonitril umkristallisiert. Weisse Kristalle. Smp.: 116°C

---

1) Hergestellt auf bekannte Weise aus Oxalsäuredimethylester und einem Ueberschuss von Aethanolamin (6 Stunden / 120°C). Smp. 169°C.

0042358

**Beispiel 10:**

**Stufe I:**

$N_1$, $N_3$-Di-n-dodecylmercaptopropionyl-diäthylentriamin.

$(n\text{-}C_{12}H_{25}SCH_2CH_2CONHCH_2CH_2\text{-})_2NH$

86,5 g n-Dodecylmercaptopropionsäure-methylester werden mit 15,5 g Diäthylentriamin unter $N_2$ und Rühren 8 Stunden auf 140°C erhitzt. Das Reaktionsprodukt wird dann durch Digerieren mit einem Gemisch von 3 Teilen Methanol und 7 Teilen Acetonitril gereinigt. Smp.: 102°C

**Stufe II:**

$N_1$, $N_3$-Di-n-dodecylmercaptopropionyl-$N_2$-(2-äthyl-n-hexyloxycarbonyl)-diäthylen-triamin

$$(n\text{-}C_{12}H_{25}SCH_2CH_2CONHCH_2CH_2\text{-})_2N\text{-}COOCH_2CH\begin{matrix} C_2H_5 \\ n\text{-}C_4H_9 \end{matrix}$$

18,5 g Stufe I-Produkt werden in 100 ml Dimethylacetamid unter Zusatz von 3,0 g Triäthylamin vorgelegt und unter Rühren mit 5,8 g Chlorameisensäure-2-äthyl-n-hexylester versetzt. (Temp.: 20-30°C). Anschliessend wird noch 4 Stunden auf 80°C erhitzt. Der Ansatz wird mit Methylenchlorid verdünnt, mit $H_2O$ ausgeschüttelt, mit $Na_2SO_4$ getrocknet und der Rückstand nach Abdampfen des Lösungsmittels aus Acetonitril/Methanol: 8/2 umkristallisiert. Weisse Kristalle. Smp.: 60°C.

**Beispiel 11:**

$$(C_{12}H_{25}SCH_2CH_2\overset{O}{\overset{\|}{C}}OCH_2)_3\text{-}CCH_2OCH_2\text{-}\underset{CH_2O\overset{\|}{C}CH_2CH_2SC_{12}H_{25}}{\overset{CH_2O\overset{O}{\overset{\|}{C}}CH_2CH_2SC_{12}H_{25}}{C}}\text{-}CH_2OCH_2C\text{-}(CH_2O\overset{O}{\overset{\|}{C}}CH_2CH_2SC_{12}H_{25})_3$$

Tripentaerylthritol-octa(3-n-dodecylthioproponate)

Ein Gemisch von 18.6 g (0.05M) Tripentaerythritol und 115.4g (0.4M) Dodecylmercaptopropionsäuremethylester wurde im Reaktionskessel

vorgelegt und auf 80°C erhitzt. Zu der Schmelze wurden 0.5g Lithiumhydrid zugegeben, und Reaktionsgemisch unter Stickstoff zuerst bei 130°C für 16 Stunden und schliesslich bei 190°C für 2 Stunden gerührt. Dieses wurde dann mit konz. HCl neutralisiert, in 200 ml Toluol aufgenommen und gut mit Wasser gewaschen, und die Lösungsmittel eingedampft. Dieses gelbbraune Oel wurde in wenig Aceton gelöst, mit 20 ml Methanol behandelt, und das Produkt bei -5°C auskristallisiert.
Fp. v. 25°C. Ausbeute: 80.0 g.

Auswendungsbeispiele

Beispiel I

100 Teile Polypropylen (Schmelzindex 2,6 g/10 Min., 230°C/2160 g) werden mit 0,3 Teilen eines in der nachstehenden Tabelle aufgeführten Additivs in einem Brabender-Plastographen bei 200° 10 Minuten geknetet. Die derart erhaltene Masse wird anschliessend in einer Plattenpresse 6 Minuten bei 260° Plattentemperatur zu 1 mm dicken Platten gespresst, aus denen Streifen von 1 cm Breite und 17 cm Länge gestanzt werden.

Die Prüfung auf Wirksamkeit der den Prüfstreifen zugesetzten Additive wird durch Hitzealterung in einem Umluftofen bei 135° und 149°C vorgenommen. Dazu werden von jeder Formulierung 3 Prüfstreifen eingesetzt. Als Endpunkt wird die beginnende, durch vollständige Versprödung leicht erkennbare Zersetzung des Prüfstreifens definiert. Die Resultate werden in Tagen angegeben.

(In allen Fällen bedeutet die Angabe in ° ("Grad") °C)

| Stabilisator Bsp. | Tage bis zur beginnenden Zersetzung bei 135°C | 149°C |
|---|---|---|
| 1 | 22 | 4 |
| 2 | 9 | 2 |
| 3 | 9 | 2 |
| 5 | 14 | 2 |
| 6 | 9 | 2 |
| 9 | 16 | 2 |
| 10 | 9 | 2 |
| 7 | 8 | 2 |
| 8 | 8 | 2 |
| 11 | 8 | 2 |

Beispiel II

100 Teile Polypropylen (Schmelzindex 2,6 g/10 Min., 230°C/2160 g) werden mit 0,3 Teilen eines in der nachstehenden Tabelle aufgeführten Additivs und 0,1 Teilen 1,3,5-Tri-(3,5-Di-t-butyl-4-hydroxybenzyl-)iso-cyanurat wie in Beispiel I homogenisiert.

| Stabilisator Bsp. | Tage bis zur beginnenden Zersetzung 135°C | 149°C |
|---|---|---|
| 1 | 247 | 65 |
| 2 | 65 | 16 |
| 3 | 53 | 13 |
| 5 | 149 | 36 |
| 6 | 124 | 28 |
| 9 | 105 | 15 |
| 10 | 72 | 19 |
| 7 | 92 | 18 |
| 8 | 69 | 14 |
| 11 | 126 | 26 |

Beispiel III

Die in Beispiel I beschriebenen Prüflinge werden überdies auf Farbstabilität geprüft, und zwar:

a) Nach Einarbeitung (Kolonne 2).

b) Nach 200 Stunden Belichtung in einem Xenotest-Gerät der Fa Hanau (Kolonne 3).

c) Nach 4-wöchiger Behandlung mit siedendem Wasser (Kolonne 4).

Für die Beurteilung wurde eine empirische Farbskala verwendet, in welcher 5 Farblosigkeit, 4 eine eben wahrnehmbare, leichte Verfärbung, 3, 2, 1 und <1 sukzessiv stärkere Verfärbung bedeuten.

| Stabilisator | Farbbeurteilung nach Skala 1-5 | | |
|---|---|---|---|
| Bsp. | Nach Einarbeitung | Nach Belichtung | siedendes Wasser 4 Wochen |
| 1 | 5 | 5 | 5 |
| 2 | 5 | 5 | 5 |
| 3 | 5 | 5 | 4-5 |
| 5 | 4-5 | 5 | 4-5 |
| 6 | 5 | 5 | 5 |
| 9 | 5 | 5 | 4-5 |
| 10 | 5 | 5 | 5 |
| 7 | 5 | 2-3 | 5 |
| 8 | 5 | 5 | 5 |
| 11 | 5 | 5 | 5 |

Beispiel IV

Die in Beispiel II beschriebenen Prüflinge werden überdies auf Farbstabilität geprüft, und zwar:

a) Nach Einarbeitung (Kolonne 2).

b) Nach 200 Stunden Belichtung in einem Xenotest-Gerät der Fa Hanau (Kolonne 3).

c) Nach 4-wöchiger Behandlung mit siedendem Wasser (Kolonne 4).

Für die Beurteilung wurde eine empirische Farbskala verwendet, in welcher 5 Farblosigkeit, 4 eine eben wahrnehmbare, leichte Verfärbung, 3, 2, 1 und <1 sukzessiv stärkere Verfärbung bedeuten.

| Stabilisator | Farbbeurteilung nach Skala 1-5 | | |
|---|---|---|---|
| Bsp. | Nach Einarbeitung | Nach Belichtung | siedendes Wasser 4 Wochen |
| 1 | 5 | 5 | 5 |
| 2 | | | |
| 3 | 5 | 5 | 4-5 |
| 5 | 5 | 5 | 4-5 |
| 6 | 5 | 5 | 5 |
| 9 | 5 | 5 | 4-5 |
| 10 | 5 | 5 | 4-5 |
| 7 | 5 | 3 | 5 |
| 8 | 5 | 5 | 5 |
| 11 | 5 | 5 | 5 |

Patentansprüche

1. Verbindungen der Formel I

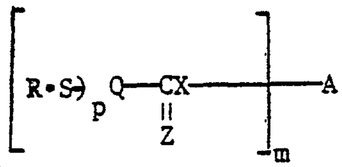 (I)

in der X einen der Reste -O-, -NH- oder $-NR^1-$ bedeutet, wobei beim Vorliegen mehrerer Reste X dieselben gleich oder verschieden sind, Z einen der Reste -O- oder -S-, m eine Zahl zwischen 1 und 12 und p eine ganze Zahl zwischen 1 und 3 bedeuten, wobei $R^1$ für einen geradkettigen oder verzweigten Alkylrest mit 1 bis 25 C-Atomen steht, in der R einen geradkettigen oder verzweigten, gesättigten oder ungesättigten Alkylrest mit 1 bis 30 Kohlenstoffatomen, welcher durch Aryl-, Aminocarbonyl-, Alkylaminocarbonyl- oder OH-Gruppen substituiert und durch eine -CO•O-, -O•CO-, -O- oder -S-Brücke unterbrochen sein kann, darstellt, in der Q einen (p+1)-wertigen geradkettigen oder verzweigten, gesättigten oder ungesättigten Kohlenwasserstoffrest mit 1 bis 20 Kohlenstoffatomen oder einen Rest der Formel 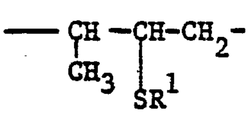 oder den 3-wertigen Rest 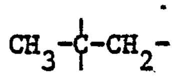 bedeutet, und

in der A

a) einen 1-wertigen Rest 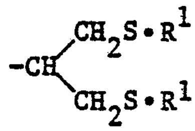 ,

oder

b) einen 2-wertigen Rest aus der Gruppe $-(C_nH_{2n}O)_q-C_nH_{2n}-$,

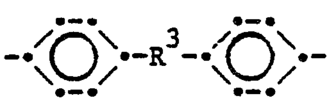, $-R^3-CO{\cdot}O-R^3-$, $-R^3-CH{=}CH-R^3-$, $-R^3-C{\equiv}C-R^3-$,

$-R^3-NH{\cdot}CO{\cdot}CO{\cdot}NH-R^3-$, 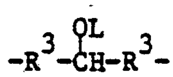, $-R^3-NH{\cdot}CO{\cdot}NH-R^3-$, 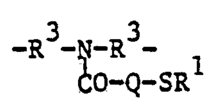 ,

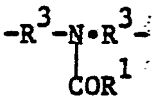 , 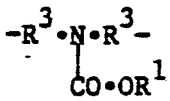 , 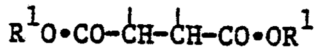, 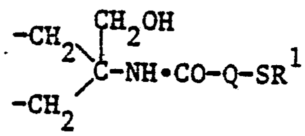,

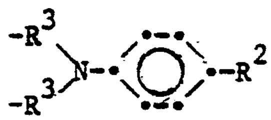 und 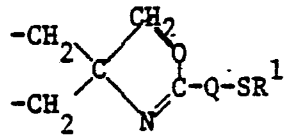,

wobei $R^2$ die Definition von $R^1$ hat, Wasserstoff bedeutet oder

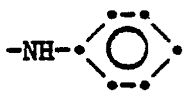 darstellt,

n eine ganze Zahl zwischen 3 und 6, q eine Zahl zwischen 1 und 20, $R^3$ einen geradkettigen oder verzweigten, gesättigten oder ungesättigten Alkylenrest mit 1 bis 20 C-Atomen und L Wasserstoff oder $-CO\cdot R^1$ bedeuten, oder

c) einen der 3-wertigen Reste

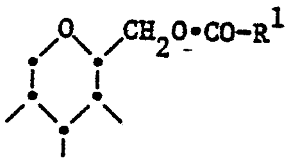 , 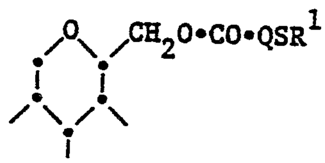 , 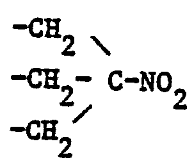

und 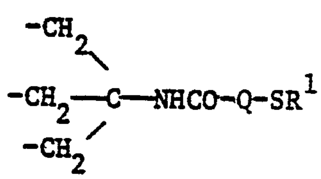

bedeutet, oder

d) einen der 4-wertigen Reste 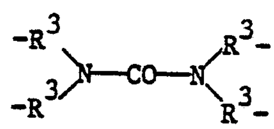 ,

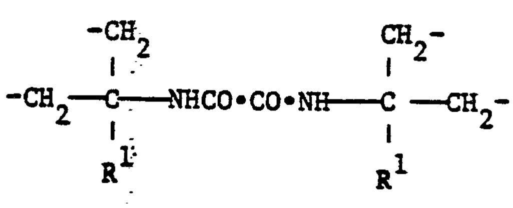 , 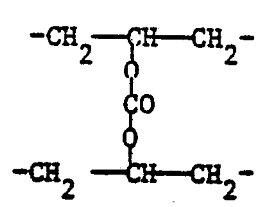 ,

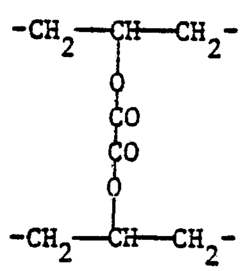

und

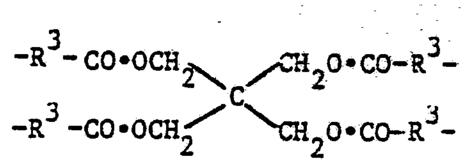

bedeutet, oder

e) einen der 6-wertigen Reste

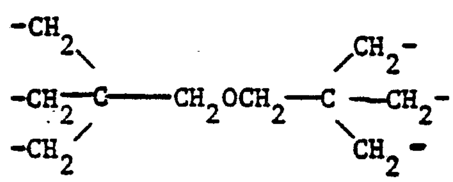

,

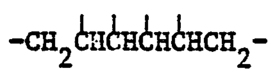

und

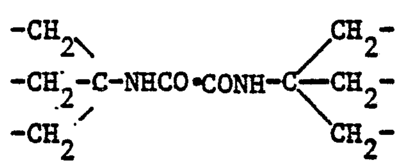

bedeutet oder

f) den 8-wertigen Rest

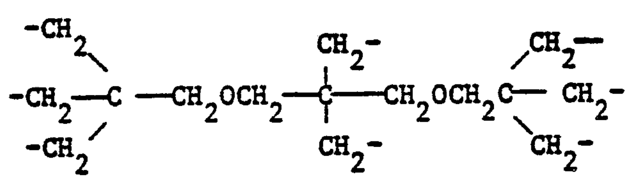

darstellt,

in der aber für den Fall, dass X einen der Reste -NH- oder $-NR^1-$ darstellt und teilweise aber nicht ausschliesslich auch -O- bedeuten kann, A auch für einen 2-wertigen, geradkettigen oder verzweigten, gesättigten oder ungesättigten Kohlenwasserstoffrest mit 2 bis 18 C-Atomen oder für einen 3- oder 4-wertigen, geradkettigen oder verzweigten, gesättigten oder ungesättigten Kohlenwasserstoffrest mit 3 bis 18C-Atomen stehen kann, und Verbindungen der Formel II

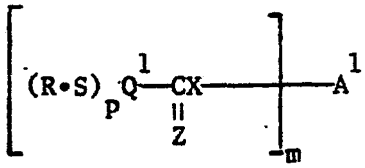 (II) ,

in der X einen der Reste -O-, -NH- oder $-NR^1-$ bedeutet, wobei beim Vorliegen mehrerer Reste X dieselben gleich oder verschieden sind, Z einen der Reste -O- oder -S-, m die Zahl 2, 3 oder 4 und p eine ganze Zahl zwischen 1 und 3 bedeuten, wobei $R^1$ für einen geradkettigen oder verzweigten Alkylrest mit 1 bis 25 C-Atomen steht, in der R einen gesättigten oder ungesättigten Alkylrest mit 1 bis 30 Kohlenstoffatomen, welcher durch Aryl-, Aminocarbonyl-, Alkylaminocarbonyl- oder OH-Gruppen substituiert und durch eine -CO•O-, -O•CO-, -O- und -S-Brücke unterbrochen sein kann, darstellt, in der $Q^1$ den Rest $-CH_2-$ oder einen Rest der Formel 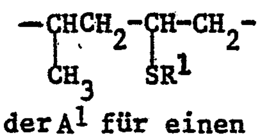 oder den 3-wertigen Rest 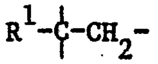 bedeutet, und in der $A^1$ für einen 2-wertigen, geradkettigen oder verzweigten, gesättigten oder ungesättigten Kohlenwasserstoffrest mit 2 bis 20 C-Atomen oder für einen 3- oder 4-wertigen, geradkettigen oder verzweigten, gesättigten oder ungesättigten Kohlenwasserstoffrest mit 3 bis 18 C-Atomen steht.

2. Verbindungen nach Anspruch 1, dadurch gekennzeichnet, dass sie der Formel I entsprechen.

3. Verbindungen nach Anspruch 1 der Formel I, in der X -O- bedeutet.

4. Verbindungen nach Anspruch 1 der Formel I, in der A

a) einen 1-wertigen Rest 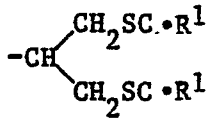 ,

oder

b) einen 2-wertigen Rest aus der Gruppe $-(C_nH_{2n}O)_qC_nH_{2n}-$,

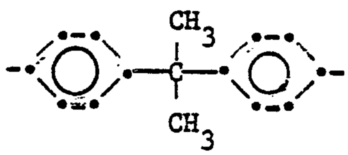 , 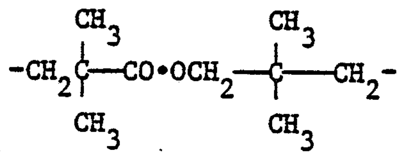 , $-CH_2-C\equiv C-CH_2-$,

$-CH_2CH=CHCH_2-$, 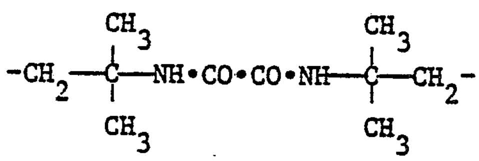 , 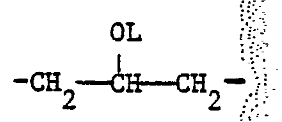 ,

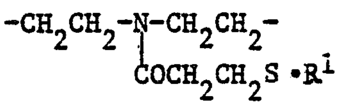 $-CH_2CH_2-NH-CO-NH-CH_2CH_2-$,

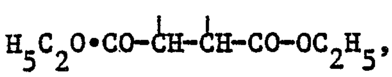, 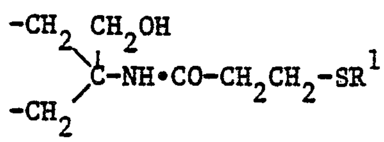 ,

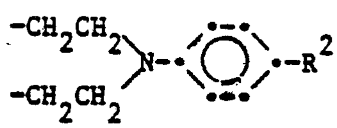 und 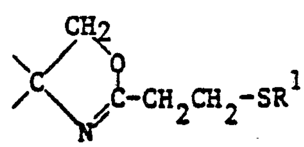,

wobei $R^2$ die Definition von $R^1$ hat, Wasserstoff bedeutet oder

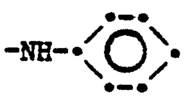 darstellt,

n eine ganze Zahl zwischen 3 und 6, q eine Zahl zwischen 1 und 20 und L Wasserstoff oder $-CO \cdot R^1$ bedeuten, oder

c) einen der 3-wertigen Reste

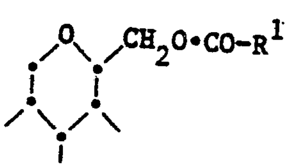 , 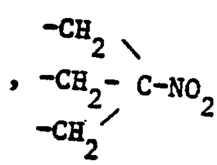 und 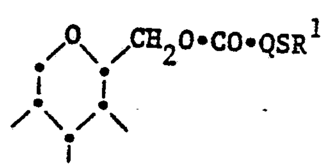

bedeutet, oder

d) einen der 4-wertigen Reste 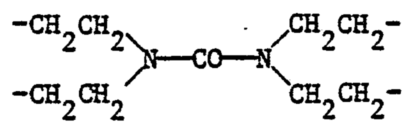 ,

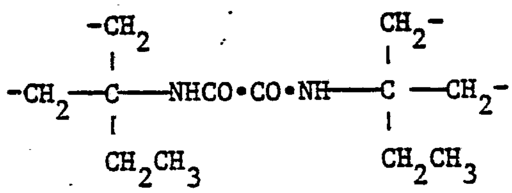 , 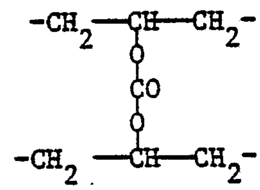 ,

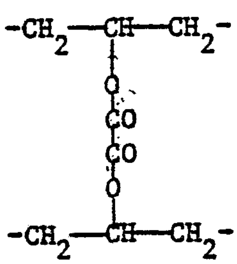 und 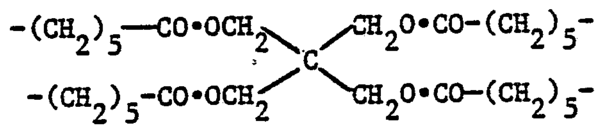

bedeutet, oder

e) einen der 6-wertigen Reste

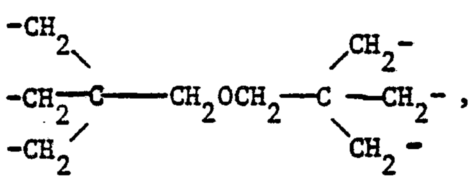 ,

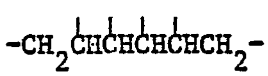 und 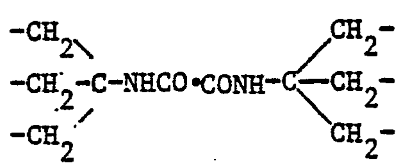

bedeutet oder

f) den 8-wertigen Rest

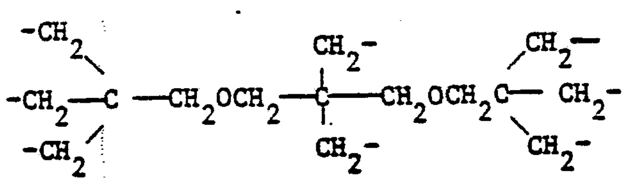 darstellt.

5. Hexa-3-n-dodecylmercapto-propionyl-dipentaerythrit als Verbindung nach Anspruch 1.

6. Verbindungen nach Anspruch 1 der Formel II, in der mindestens ein Rest X -O- bedeutet.

7. Verwendung von Verbindungen nach Anspruch 1 als Stabilisatoren oder Co-Stabilisatoren für organisches Material, wobei vorzugsweise die Konzentration der erfindungsgemässen Verbindungen 0,005 bis 10 Gew.-%, insbesondere 0,05 bis 1,5 Gew.-%, bezogen auf die gesamte stabilisierte Mischung, beträgt.

8. Verwendung nach Anspruch 7, dadurch gekennzeichnet, dass die Verbindung nach Anspruch 1 zusammen mit phenolischen Stabilisatoren eingesetzt werden.

9. Verwendung nach Anspruch 7 in organischen Polymeren, insbesondere in Polyolefinen.

10. Verwendung nach Anspruch 7 in Schmiermitteln.

11. Durch Verbindungen nach Anspruch 1 oder durch Gemische von Verbindungen nach Anspruch 1 mit phenolischen Stabilisatoren stabilisiertes organisches Material.

0042358

Europäisches Patentamt

# EUROPÄISCHER RECHERCHENBERICHT

Nummer der Anmeldung

EP 81 81 0232

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| X | US - A - 4 080 364 (O.S. KAUDER et al.)<br>* Spalte 6, Zeile 2; Spalte 27, Beispiel XXII *<br>-- | 1-5 | C 07 C 149/23<br>149/20<br>C 07 D 263/14<br>C 08 K 5/36 |
| X | BE - A - 856 674 (ANGUS)<br>* Seite 4, Beispiel 4 *<br>-- | 1-4 | |
| X | CHEMICAL ABSTRACTS, Band 89, Nr. 20, 13. November 1978, Seite 36, Nr. 164435j<br>Columbus, Ohio, U.S.A.<br>& JP - A - 78 73241 (ADEKA ARGUS CHEMICAL CO., LTD.) 29-06-1978<br>* Zusammenfassung *<br>-- | 1-5 | RECHERCHIERTE SACHGEBIETE (Int. Cl.³)<br>C 07 C 149/23<br>149/20<br>C 07 D 263/14<br>C 08 K 5/36 |
| DX | US - A - 3 758 549 (MARTIN DEXTER et al.)<br>* Spalten 8,9; Beispiel 2 *<br>-- | 1-4 | |
| PX | US - A - 4 216 115 (MILLER)<br>* Spalte 3, Tafel I; Beispiel 5 *<br>---- | 1-4 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |

X Der vorliegende Recherchenbericht wurde für alle Patentansprüche erstellt.

| Recherchenort | Abschlußdatum der Recherche | Prüfer |
|---|---|---|
| Den Haag | 21-09-1981 | GRAMAGLIA |